# EUROPEAN PATENT SPECIFICATION

(11) **EP 3 936 039 B1**
(45) Date of publication and mention of the grant of the patent: **20.08.2025**
(21) Application number: 20767374.0
(22) Date of filing: 04.03.2020
(51) Int. Cl.: A61B 5/256, A61B 5/27, A61B 5/282

(54) **TOOL FOR ACQUIRING BIOLOGICAL SIGNAL**
WERKZEUG ZUR ERFASSUNG VON BIOLOGISCHEN SIGNALEN
OUTIL D'ACQUISITION DE SIGNAL BIOLOGIQUE

(30) Priority: 05.03.2019 JP 2019039781
(43) Date of publication of application: 12.01.2022
(73) Proprietor: Toray Industries, Inc., Tokyo 103-8666 (JP)
(72) Inventor: TAKARADA, Hiromi, Tokyo 103-8666 (JP); MATSUO, Ryo, Tokyo 103-8666 (JP); ITAGAKI, Ichiro, Tokyo 103-8666 (JP)
(74) Representative: Mewburn Ellis LLP
(86) International application number: PCT/JP2020/009263
(87) International publication number: WO 2020/179845

(56) References cited:
- WO-A1-2017/013995
- JP-A- 2013 085 575
- JP-A- 2018 175 388
- US-A1- 2005 275 416
- US-A1- 2006 117 805

## Description

### Field

The present invention relates to a biosignal acquiring tool.

### Background

Human biosignals provide useful information for observing the health condition and active state of a person, making a diagnosis of a disease of the person, or estimating the mental condition of the person. Examples of biosignals include heartbeats which are biosignals from a human heart and an electrocardiogram. In order to acquire such biosignals, there is known a technique of measuring a potential difference by attaching an electrode to a human body.

In the medical field, generally, an electrocardiogram is acquired by attaching an electrode connected to a device via a cord to a human body. In this case, a subject cannot leave the place and is restricted in activity.

In contrast, as a method for acquiring biosignals for long hours, there is known Holter electrocardiography in which, with an electrode being attached to a human body together with a small-sized device, an electrocardiogram is carried out in everyday life. However, in Holter electrocardiography, an adhesive sheet for attaching the electrode causes a skin rash easily, and is sometimes accompanied by itching. Furthermore, it is difficult to reattach the electrode to an appropriate attachment portion, and therefore, the inconvenience of, for example, not being allowed to take a bath during measurement is involved.

There are also known heartbeat sensors configured to monitor heart beats in order to provide efficient sports training and check the safety. The developments of belt-type and shirt-type heartbeat sensors equipped with a small-sized device and an electrode are variously proceeding. In particular, belt-type heartbeat sensors are easier to wear and allow size adjustment, and therefore, a belt-type heartbeat sensor in which an electrode is attached to the inside of an elastic belt has been widely used. However, the belt wound around a chest often causes a noise due to active activities and perspiration, or often slips down to cause a measurement failure. In addition, when the belt is tightly fastened in order to prevent the belt from slipping down, severe discomfort is caused by pressure, so that the belt-type heartbeat sensor cannot be used for long hours.

Under such circumstances, a belt that prevents noise generation during biosignal acquisition and resists slipping down has been studied. For example, Patent Literature 1 discloses a configuration in which a belt is connected inside right and left electrodes, whereby the belt presses down components including the electrodes from above. This configuration allows the electrodes to be kept in contact with the skin of a user even during vigorous motions. Note that Patent Literature 1 discloses that a base unit including the electrodes and a belt fixing part is preferably flexible, and accordingly is produced by an elastic material, such as rubber, that can more easily bend during the use of the belt.

Patent Literature 2 discloses that an elastic strap and a hard coupling member are connected to each other, and a device body including an electrode is detachably attached to the coupling member. According to Patent Literature 2, it is believed that adhesion of a biological information detecting device to a human body can be enhanced by the strap, and also an external tensile force of the strap is not transmitted to the device body including wiring and accordingly neither disconnection nor noise generation is likely to occur.

However, elastic materials, such as rubber and plastics, disclosed as materials for components including the electrodes in the above-mentioned patent literatures bend along a human body, whereby adhesion is achieved, whereas the elastic materials are neither air-permeable nor water absorptive, so that the skin easily gets sweaty, and when a wearer sweats, sweat is accumulated between the components and the skin, and as a result, not only a feeling of discomfort is given, but also the belt becomes slippery and the electrodes are displaced, whereby noise generation occurs.

Furthermore, according to Patent Literature 2, when the electrode is pressed against a skin, a connector part connected to the detecting device also closely adheres to the skin, and as a result, the skin gets sweaty and unpleasantness is felt, and furthermore, the vibration of the electronic device is easily transmitted to electrode components.

As a technique for preventing a baseband completely wound around a chest from being displaced, Patent Literature 3 discloses a technique in which a band obliquely wound via a shoulder is provided and an electrode is disposed on the band. Patent Literature 3 states that a projecting part, such as a pad or a sponge, is provided on a skin-side surface of the band and an electrode is disposed thereon, whereby the electrode can be stably in contact with a recession part of the body. However, since the projecting part is directly disposed on the band, biosignals can be stably acquired when the body remains stationary, but, when the body is moving, the band is pulled or vibrates due to the body movement, whereby the electrode is displaced, so that noise generation inevitably occurs.

Patent Literature 4 discloses a technique related to an elastic garment equipped with an electrical conductive part. According to this technique, it is believed that a cover is provided in order to press the electrical conductive part provided in a body fabric against a skin, whereby, when a body is moving, an electrode can be kept in contact with the skin. However, even when the body fabric is an elastic material, as long as the body fabric covers half the body of a wearer as a garment, the body fabric is pulled due to a large body movement, whereby the body fabric is inevitably displaced. Furthermore, the electrode is disposed on the body fabric, and the body fabric continuously surrounds the body as a garment, and therefore, the electrode cannot be separated from the displacement of the body fabric due to the body movement. Patent Literature 4 also states that a non-slip material made of elastic yarn or resin is provided in the surroundings of the electrode. However, as long as, like the electrode, this non-slip material is present, on the body fabric of the garment, when the displacement of the body fabric is caused by a body movement exceeding the elasticity of the body fabric, the electrode is inevitably displaced. Patent Literatures 5 and 6 disclose further means of acquiring biosignals.

As described above, in the well-known art, it has been difficult to acquire biosignals for long hours without giving a feeling of pressure to a human body.

### Citation List

### Patent Literature

Patent Literature 1: JP 2006-187615; Patent
Literature 2: JP 2014-13267; Patent Literature 3: JP 2015-77226; Patent Literature 4: JP 2017-31534; Patent
Literature 5: US 2006/0117805 A1; Patent Literature 6: US 2005/0275416 A1

### Summary

### Technical Problem

The present invention has been devised in view of the above-described circumstances, and an object of the present invention is to provide a biosignal acquiring tool capable of acquiring biosignals for long hours without giving a feeling of pressure to a human body.

### Solution to Problem

To solve the problem described above and to achieve the object, a biosignal acquiring tool according to the present invention includes: a textile structure in which a non-slip textile is disposed; an electrode positioned on a back side of the textile structure; a connector configured to connect an electronic device configured to acquire a signal from the electrode; and an elastic belt capable of being stretched independently of the textile structure, the elastic belt being disposed in parallel with the textile structure to face a front side of the textile structure that is different from a back side on which the electrode is positioned, the elastic belt being coupled to the textile structure at a portion different from a portion at which the electrode is positioned and at a portion different from surroundings of the portion, the textile structure being capable of being displaced within a range of 2 mm or more and 10 mm or less in a width direction of the elastic belt, and wherein the back side corresponds to a side closer to a wearer's skin in use.

In the biosignal acquiring tool according to the present invention, the non-slip textile is a knitted fabric including an elastic fiber and an inelastic fiber, and has a surface occupancy of the elastic fiber being 30% or more and 70% or less.

In the biosignal acquiring tool according to the present invention, the textile structure has a tubular shape, and the elastic belt is configured to pass through inside of the tubular shape.

In the biosignal acquiring tool according to the present invention, the textile structure includes a cushioning member having a thickness of 2 to 10 mm, the cushioning member being disposed outside the electrode and inside the elastic belt disposed in parallel with the textile structure.

In the biosignal acquiring tool according to the present invention, the textile structure and the elastic belt are integrally fixed between the connector and the electrode.

In the biosignal acquiring tool according to the present invention, the electrode is removable, and fixed to the textile structure with a metal hook.

In the biosignal acquiring tool according to the present invention, the electrode is configured with an electrical conductive textile including a nanofiber.

The biosignal acquiring tool according to the present invention further includes a waterproof cover configured to cover an upper portion and a front-side surface of the electronic device from a back-side surface of the connector, with the electronic device being attached to the connector.

### Advantageous Effects of Invention

According to the present invention, biosignals can be acquired for long hours without giving a feeling of pressure to a human body.

### Brief Description of Drawings

FIG. 1 is a perspective view of a configuration of the front side of a biosignal acquiring tool according to an embodiment of the present invention.
FIG. 2 is a perspective view of a configuration of the back side of the biosignal acquiring tool according to the embodiment of the present invention.
FIG. 3 is a sectional view taken along line A-A in FIG. 2.
FIG. 4 is a perspective view of a configuration of a biosignal acquiring tool according to a first modification of the embodiment of the present invention.
FIG. 5 is a sectional view taken along line B-B in FIG. 4.
FIG. 6 is a perspective view of a configuration of a biosignal acquiring tool according to a second modification of the embodiment of the present invention.
FIG. 7 is a perspective view of a configuration of a biosignal acquiring tool according to a third modification of the embodiment of the present invention.
FIG. 8 is a perspective view of a configuration of a biosignal acquiring tool according to a fourth modification of the embodiment of the present invention.
FIG. 9 is a perspective view of a configuration of a biosignal acquiring tool according to a fifth modification of the embodiment of the present invention.
FIG. 10 is a diagram illustrating electrocardiogram data acquired by an electronic device in Example 1.
FIG. 11 is a perspective view of a configuration of a biosignal acquiring tool used in Comparative Example 1.
FIG. 12 is a diagram illustrating electrocardiogram data acquired by an electronic device in Comparative Example 1.
FIG. 13 is a diagram illustrating electrocardiogram data acquired by an electronic device in Comparative Example 2.

### Description of Embodiments

Hereinafter, an embodiment of the present invention (hereinafter, referred to as "the embodiment") will be described with reference to the accompanying drawings. Note that the drawings are merely schematic drawings.

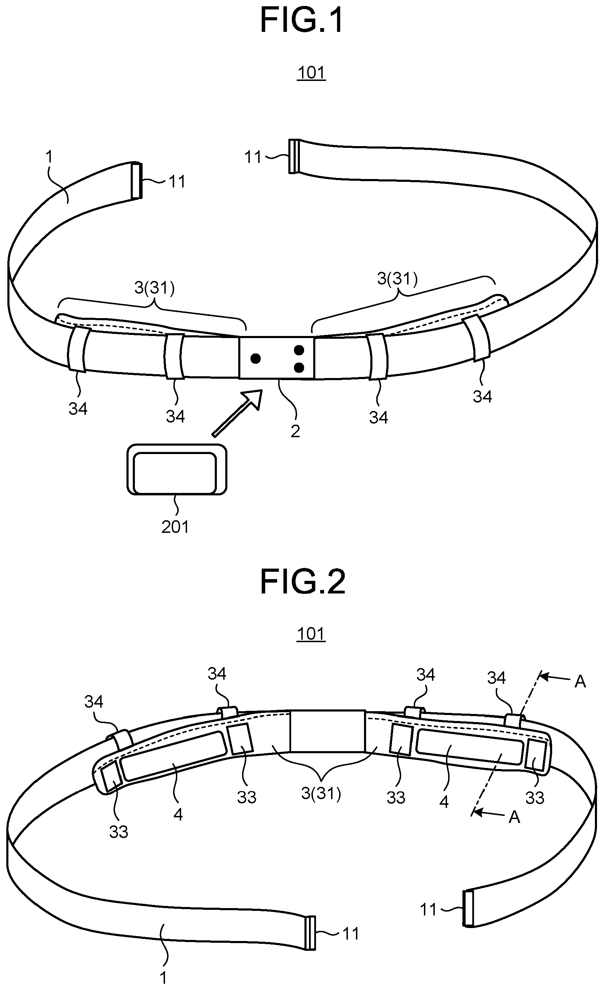

FIG. 1 and FIG. 2 are perspective views of configurations of the front side and the back side of a biosignal acquiring tool according to the embodiment of the present invention, respectively. The front side and the back side mentioned herein correspond to the outer side and the inner side (a side closer to the skin of a human body) of the biosignal acquiring tool, respectively, when the biosignal acquiring tool is worn by the human body. The biosignal acquiring tool 101 illustrated in FIG. 1 and FIG. 2 includes an elastic belt 1, a connector 2, a textile structure 3, and an electrode 4.

The elastic belt 1 is band-shaped, and each end of the elastic belt 1 has a detachable/attachable belt joint 11. The elastic belt 1 contains an elastic body, such as polyurethane or rubber, and is configured using what is called woven rubber or knitted rubber with a tape-shaped form of fibers. The elastic belt 1 faces the front side of the textile structure 3, and is configured to press the electrode 4 on the skin of the human body when the biosignal acquiring tool 101 is worn by the human body. On the assumption of wearers having various body shapes, the elastic belt 1 may be provided with, for example, a ring or a fastener for length adjustment. In this case, if a mark indicating an appropriate adjustment position is put on the elastic belt 1 as a guide for adjusting the length so as to be fitted to the circumferential length of the body of a wearer, the length of the elastic belt 1 can be easily adjusted so that both measurement with less noises and belt fastening with comfortable wearing can be achieved.

The connector 2 is positioned at the center of the front side of the biosignal acquiring tool 101, and an electronic device 201 configured to acquire biosignals is attached to the connector 2. The configuration of the connector 2 is not particularly limited as long as the connector 2 can be electrically continuous with the electronic device 201, and, for example, a metal button configured to be fixed to the textile structure 3 by caulking can be used. The connector 2 with such configuration allows the electronic device 201 to be easily detached and attached, and can be easily fixed to soft textiles.

The textile structure 3 is configured using a textile made of a knitted or woven fabric. The textile is water absorptive and air-permeable, and therefore further reduces a sweaty feeling, compared to a flat surface of a plastic molded article such as rubber or a film. Furthermore, the textile reasonably absorbs sweat, and therefore, unlike a plastic flat surface, sweat does not accumulate on the textile, so that the textile does not become slippery.

The electrode 4 is disposed on the back side (a side closer to a skin) of the textile structure 3. The electrode 4 is fixed to the textile structure by being sewn on the textile structure or being pasted on the textile with an adhesive, and is electrically connected to the electronic device 201 via the connector 2 and wiring not illustrated. The wiring is not exposed at a surface of the biosignal acquiring tool 101.

The electrode 4 is configured using an electrical conductive textile. This configuration prevents liquid sweat of a human body from being accumulated on the electrode 4, and thus reduces displacement due to a body movement during perspiration. When a material obtained by impregnating nanofiber with an electrical conductive substance is used as the electrical conductive textile, the nanofiber itself is resistant to slipping on the skin, and therefore, the electrical conductive textile is more resistant to displacement caused by a body movement.

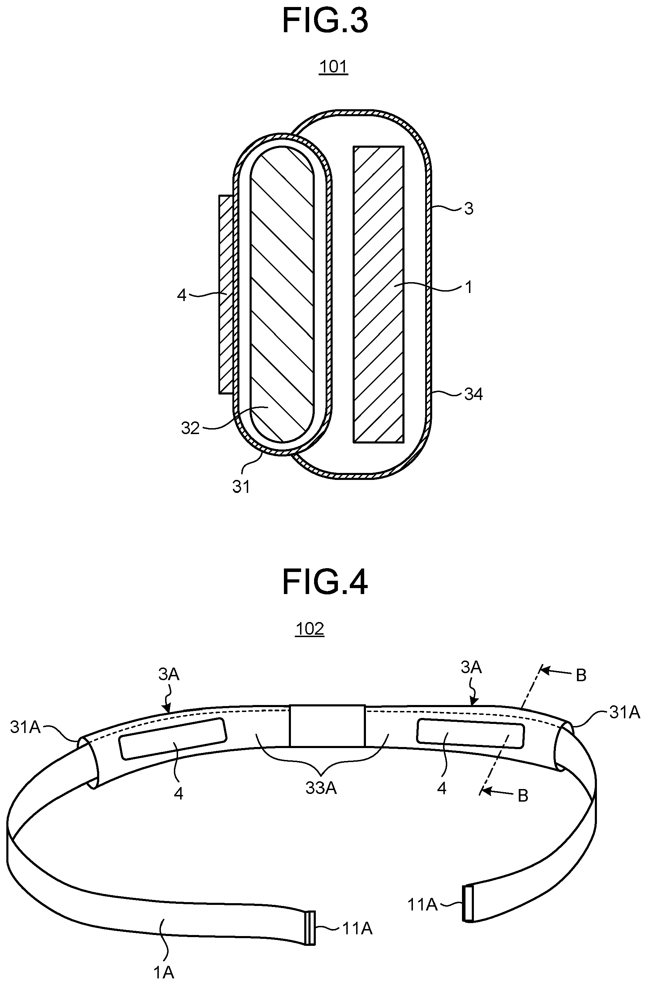

FIG. 3 is a sectional view taken along line A-A in FIG. 2. As illustrated in FIG. 3, the textile structure 3 includes: a main body 31 having a tubular shape; and a cushioning member 32 provided inside the main body 31. The cushioning member 32 is not fixed to the elastic belt 1, so that the cushioning member 32 does not follow a movement of the elastic belt 1. Thus, the cushioning member 32 has the effect of substantially preventing a movement of the elastic belt 1 from being transmitted to the electrode 4 pressed against a skin.

A non-slip textile 33 is stuck on the perimeter of the electrode 4 of the textile structure 3, in which the non-slip textile 33 is a knitted fabric including an elastic fiber and an inelastic fiber and the surface occupancy of the elastic fiber is 30% or more and 70% or less. When the surface occupancy of the elastic fiber is 30% or more, non-slip properties belonging to the elastic fiber is achieved with significance. The surface occupancy of the elastic fiber is more preferably 50% or more because better non-slip performance can be achieved. When the surface occupancy of the elastic fiber is 70% or less, stability as a textile can be achieved.

The textile structure 3 is caught in the skin of a wearer and accordingly does not move, whereby the electrode is fixed, and noise due to a body movement is less likely to be caused during biosignal acquisition, compared to a textile structure having a surface occupancy of the elastic fiber lower than the above-mentioned surface occupancy. Furthermore, the non-slip textile 33 is more water absorptive and more air-permeable than a non-slip material such as a rubber molded article, and therefore is less likely to cause a sweaty condition and substantially prevents sweat from being accumulated on the surface. Therefore, non-slip performance does not decrease too much even during perspiration, and the electrode 4 can be fixed to the skin.

The non-slip textile 33 is disposed on a side, closer to the skin, of the textile structure 3, and the outer surface of the non-slip textile 33, the outer surface being in contact with the elastic belt 1 and a garment, preferably has the poorest possible non-slip properties. The reason for this is that, if the outer surface has excellent non-slip properties, the non-slip textile 33 is caught by friction against movements of the elastic belt 1 and the garment, and transmits the movements to the electrode 4. The non-slip textile 33 in which the elastic fiber is exposed in large amounts is less likely to be displaced from the skin, and furthermore, perspiration does not cause to be accumulated at an interface between the non-slip textile 33 and the skin, and therefore, non-slip properties do not decrease.

The textile structure 3 is disposed in parallel with the elastic belt 1 in the longitudinal direction of the elastic belt 1. This can substantially prevent a noise increase caused by the direct application, to the textile structure 3, of a tensile stress applied in a state in which a human body wears the elastic belt 1. Furthermore, the textile structure 3 is configured to be in parallel with the elastic belt 1, and the electrode 4 is configured to come into contact with the skin side of the textile structure 3, so that the electrode 4 is pressed against the skin by the elastic belt 1. As a result, the impedance of an interface between the electrode 4 and the skin decreases, so that biosignal acquisition performance is enhanced.

The textile structure 3 is fixed to the elastic belt 1 only at the boundary between the elastic belt 1 and the connector 2, whereas the textile structure 3 is coupled to the elastic belt 1 so that the textile structure 3 can be displaced in the width direction of the elastic belt 1. Specifically, the textile structure 3 is coupled to the elastic belt 1 so that the textile structure 3 can move upward and downward within a range of 2 mm or more and 10 mm or less in the width direction of the elastic belt 1. Of the textile structure 3, a part in which the electrode 4 is provided and the surroundings of the part are not fixed to the elastic belt 1. Hereinafter, such coupling structure between the elastic belt 1 and the textile structure 3 is sometimes referred to as a flexible structure. The textile structure 3 having such configuration is less likely to be directly influenced by the displacement of the elastic belt 1 affected by a change in the circumferential length of a human body and vibration due to breathing or a body movement, and accordingly signal noise is reduced and measurement accuracy is enhanced.

In one embodiment of the present invention, a belt loop 34 through which the elastic belt 1 passes is attached to the textile structure 3. The belt loop 34 is made from a soft material, and has a larger section than the elastic belt 1. Thus, when the elastic belt 1 passes through the belt loop 34, a little slack is allowed in the elastic belt 1, and therefore, even when the elastic belt 1 is stretched or displaced by a body movement, the textile structure 3 remains stuck on the skin, and accordingly is less affected by the displacement.

When the textile structure 3 has a thick cushioning member, a force with which a body is pressed by the elastic belt 1 concentrates on the electrode 4, and thus, even when the force is weak, the electrode 4 can be sufficiently pressed against the body. Examples of a material for this cushioning member include, but not limited to, soft urethane, rubber sponge, and polyethylene foam, but it is important that the thickness of the cushioning member is 2 to 10 mm. When the thickness is 2 mm or more, the structure can be efficiently pressed against the skin by the elastic belt 1. When the thickness is 10 mm or less, the textile structure 3 does not protrude greatly from the body, and therefore, the textile structure 3 is pushed by external force and moved, and thus the problem of noise generation in the electrode 4 is reduced.

The elastic belt 1 and the textile structure 3 are preferably separated from and disposed in parallel with each other until reaching the closest possible point to the connector 2. The weight of the electronic device 201 is applied to the connector 2, and accordingly the connector 2 is easily displaced upward and downward due to a body movement. However, the elastic belt 1 supports the load of the electronic device 201, while the load of the electronic device 201 is not applied to the textile structure 3 having a deformable flexible structure and being connected to the connector, so that the electrode 4 is not affected by the displacement due to the body movement.

The biosignal acquiring tool according to the above-described embodiment of the present invention includes: a textile structure in which a non-slip textile is disposed; an electrode positioned on a surface of the textile structure; a connector configured to connect an electronic device configured to acquire a signal from the electrode; and an elastic belt capable of being stretched independently of the textile structure. The elastic belt is disposed in parallel with the textile structure to face a front side different from a back side on which the electrode of the textile structure is positioned, and the elastic belt is coupled to the textile structure at a portion different from a portion at which the electrode is positioned and at a portion different from surroundings of the portion. The textile structure is capable of being displaced within a range of 2 mm or more and 10 mm or less in the width direction of the elastic belt, so that biosignals, such as heart beats and an electrocardiogram, can be acquired for long hours without giving a feeling of pressure to a human body.

According to the present embodiment, a cushioning member and a non-slip textile are used, and furthermore, a belt and an electrode component are separate members and the electrode is not fixed onto the belt, so that a body movement has a smaller influence, and furthermore, the effective elastic length of the elastic belt can be ensured to be sufficiently long, so that the elastic belt can be fastened around a human body without giving a feeling of intense pressure. Thus, there can be provided a biosignal measurement tool that allows wearers having different body shapes to comfortably and easily acquire highly accurate biological information without a feeling of intense pressure and a sweaty feeling in their everyday lives involving body movements, such as walking and going up and down the stairs.

Furthermore, according to the present embodiment, the elastic belt wound around a chest and the structure including the electrode are disposed in parallel with each other, and accordingly the tensile stress of the belt in the longitudinal direction is not applied to the structure itself, so that, even when the belt vibrates or is stretched due to a body movement, the electrode is not affected thereby. Furthermore, the electronic device attached to the connector transmits vibration due to a body movement under its own weight to the belt, but the configuration in which the electrode and the belt are disposed in parallel with each other has the effect of not causing the weight to directly affect adhesion of the electrode to the skin.

### (Modifications)

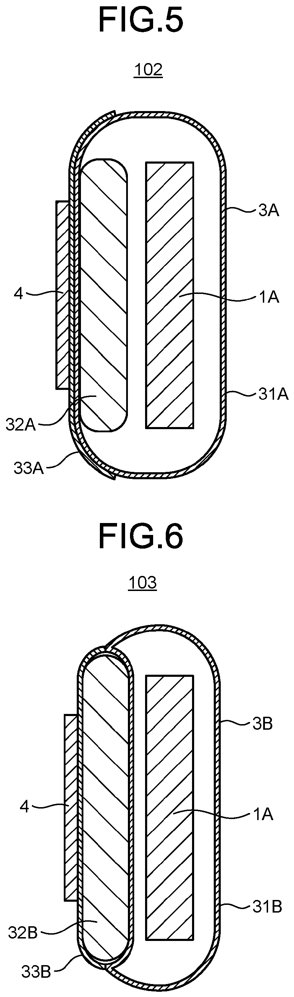

FIG. 4 is a perspective view of a configuration of a biosignal acquiring tool according to a first modification. FIG. 5 is a sectional view taken along line B-B in FIG. 4. A biosignal acquiring tool 102 illustrated in these figures includes: an elastic belt 1A including a belt joint 11A; a textile structure 3A having a tubular shape; and an electrode 4 positioned on the back side of the textile structure 3A. The textile structure 3A includes: a main body 31A having a tubular shape; a cushioning member 32A stuck on an inner surface of the tubular shape of the main body 31A, the inner surface being positioned between the elastic belt 1A and the electrode 4; and a non-slip textile 33A stuck on a portion on the back side of the outer surface of the tubular shape of the main body 31A. The electrode 4 is stuck on the non-slip textile 33A. The elastic belt 1A passes through the inside of the main body 31A, whereby, without using the belt loop as illustrated in FIG. 1, the electrode 4 can be disposed at an appropriate position inside the elastic belt 1A, and also the flexible structure allows the belt and the electrode to be indirectly coupled to each other. Since the non-slip textile 33A is disposed on the back side of the textile structure 3A, the non-slip textile 33A is fixed to the skin so that the electrode 4 is less likely to be displaced from the skin, and furthermore, the non-slip textile 33A is not caught in the elastic belt 1A, and, as a result, the displacement of the elastic belt 1A is not transmitted to the electrode 4. Hence, according to the first modification, noise generation due to a body movement can be reduced.

FIG. 6 is a perspective view of a configuration of a biosignal acquiring tool according to a second modification. A biosignal acquiring tool 103 illustrated in FIG. 6 has the same appearance as that of the biosignal acquiring tool 102 illustrated in FIG. 4, but a section of the biosignal acquiring tool 103 corresponding to the section taken along line B-B in FIG. 4 is different from that of the first modification. The biosignal acquiring tool 103 includes: an elastic belt 1A; a textile structure 3B having a tubular shape; and an electrode 4 positioned on the back side of the textile structure 3B. The textile structure 3B includes: a main body 31B having a tubular shape; a cushioning member 32B stuck on a portion on the back side of the outer surface of the tubular shape of the main body 31B; and a non-slip textile 33B stuck on a portion on the back side of the surface of the cushioning member 32B (a portion opposite to a surface stuck on the main body 31B). The electrode 4 is stuck on the non-slip textile 33B. In this case, the same effects as those obtained in the first modification can be obtained.

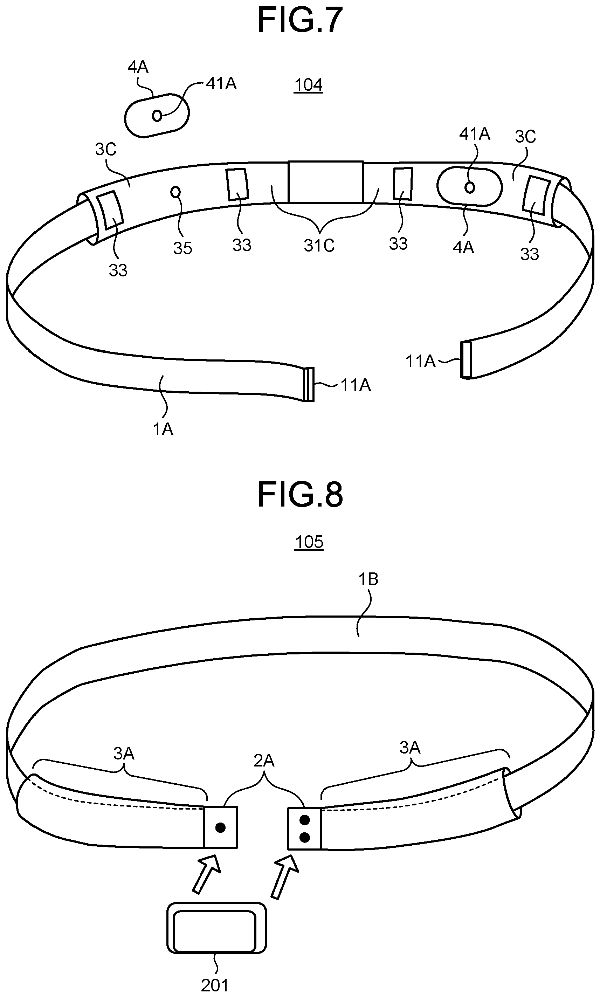

FIG. 7 is a perspective view of a configuration of a biosignal acquiring tool according to a third modification. A biosignal acquiring tool 104 illustrated in FIG. 7 includes: an elastic belt 1A; a textile structure 3C having a tubular shape; and an electrode 4A detachably attached to the textile structure 3C. A metal button 35 electrically connected to internal wiring is provided in a portion on the back side of the outer surface of the tubular shape of the textile structure 3C. Furthermore, a metal button 41A capable of being fitted to the button 35 of the textile structure 3C is provided on a surface of the electrode 4A. Like the textile structure 3A or 3B, the textile structure 3C includes a main body 31C and a cushioning member not illustrated. In the biosignal acquiring tool 104 having such a configuration, the electrode 4A can also be removed from the textile structure 3C, and therefore, an electronic device 201 and the electrode 4A can be removed for washing. Furthermore, the electrode 4A is suitably replaced, so that satisfactory biosignals can be acquired at all times. Furthermore, a non-slip textile 33 can be disposed in a part on the back side of the textile structure 3C having a tubular shape.

FIG. 8 is a perspective view of a configuration of a biosignal acquiring tool according to a fourth modification. A biosignal acquiring tool 105 illustrated in FIG. 8 includes: an elastic belt 1B; a connector 2A; and a textile structure 3A. Both ends of the elastic belt 1B are connected to the connector 2A. The connector 2A is separated into portions, and, when the electronic device 201 is attached to the connector 2A, the separated portions of the connector 2A are electrically continuous, and furthermore, the biosignal acquiring tool 105 and the electronic device 201 are continuous to make a closed form. Thus, a joint is unnecessary in the elastic belt 1B, so that the biosignal acquiring tool 105 has a simpler structure. Furthermore, a wearer only attaches the electronic device 201 to the connector 2A in the front of the body to complete wearing the biosignal acquiring tool 105, and thus the wearing is easy.

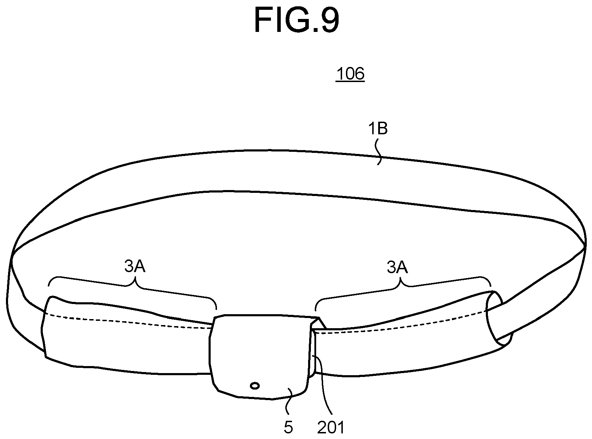

FIG. 9 is a perspective view of a configuration of a biosignal acquiring tool according to a fifth modification. A biosignal acquiring tool 106 illustrated in FIG. 9 includes: an elastic belt 1B; a connector 2A; a textile structure 3A; and a waterproof cover 5. Although the connector 2A is hidden behind the waterproof cover 5 and the electronic device 201 in FIG. 9, the configuration is the same as that illustrated in FIG. 8. The waterproof cover 5 is configured to cover an upper portion and a front-side surface of the electronic device 201 from a back-side surface of the connector 2A configured to connect the electronic device 201. In the case where a user heavily sweats during biosignal acquisition, the waterproof cover 5 prevents an electronic device 201 attached to the elastic belt 1B and the connector 2A from getting wet with sweat pouring down the skin inside a garment. This configuration can prevent the electronic device 201 and the connector 2A from getting wet and thereby causing electrical conduction, and thus can prevent a failure in biosignal measurements. In order to avoid liquid, such as sweat, from flowing into the connector 2A, it is required to accommodate the electronic device 201 and the connectors 2A inside the waterproof cover 5. For this purpose, the waterproof cover 5 is preferably provided on the elastic belt 1B, not on a main body of the electronic device 201. Examples of a material for the waterproof cover 5 include, but not limited to, cloth, a film, and a resin molded article. The waterproof cover 5 directly touches a user's skin, and therefore is preferably made from cloth rather than a hard resin or a film.

As a method for giving waterproofness to a cloth, any method can be employed. Examples of the method include: sticking a film capable of thermocompression-bonding on a surface of a cloth; coating a cloth with a urethane resin; and sewing a waterproof coating cloth and a cloth having no waterproofness together. Alternatively, a part of the waterproof cover 5 may be fixed to the elastic belt 1B with a snap button or the like so that the waterproof cover 5 does not come off the electronic device 201 due to friction or the like with a garment. Alternatively, the waterproof cover 5 may be made into a tubular shape, and a part of the waterproof cover 5 may be sewn on the elastic belt 1B, and the electronic device 201 may be inserted into the inside of the waterproof cover 5 from side and attached to the connector 2A.

### [Examples]

Hereinafter, examples of the present invention will be described. Note that the present invention is not limited by the examples described below.

### (Example 1)

In Example 1, an elastic belt having a configuration illustrated in FIG. 1 and FIG. 2 was produced, and biosignals were acquired. As an electronic device, Transmitter 01 (manufactured by NTT DOCOMO, INC.) was used. The thickness of a cushioning member built in a textile structure was 5 mm.

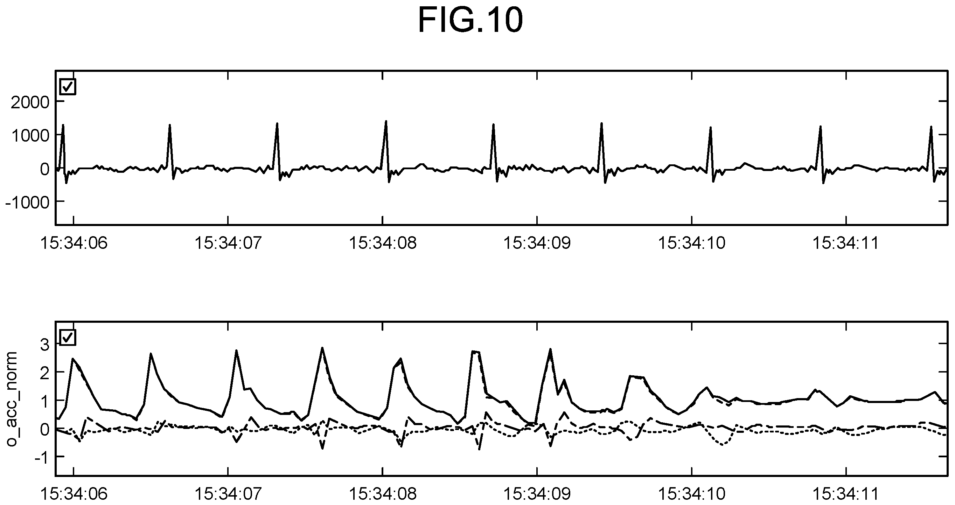

A wearer jogged for 5 minutes, then took a rest for 2 minutes, then went up and down the stairs for 2 minutes, and then kept wearing the elastic belt for 8 hours. FIG. 10 is a diagram illustrating electrocardiogram (ECG) waveform data acquired by Transmitter 01 when the wearer went up and down the stairs in Example 1.

### (Comparative Example 1)

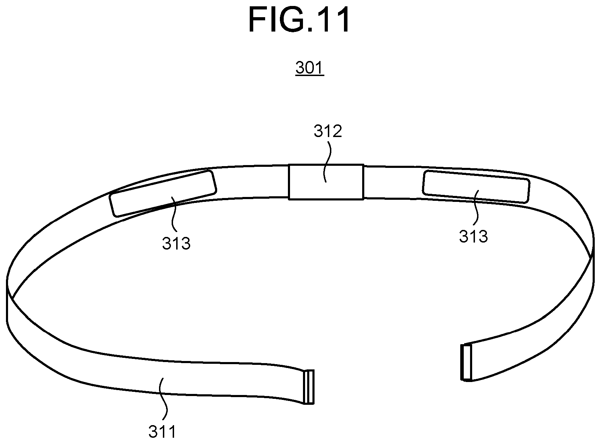

In Comparative Example 1, using a biosignal acquiring tool illustrated in FIG. 11, heart beats were acquired in the same manner as in Example 1. A biosignal acquiring tool 301 illustrated in FIG. 11 includes: an elastic belt 311; a connector 312; and electrodes 313 directly fixed to the back side of the elastic belt 311, in which wiring is provided between the connector 312 and the electrodes 313. Thus, the electrodes 313 and the wiring stuck inhibit the elastic belt 311 from stretching in an area from an end of one of the electrodes 313 to an end of another one of the electrodes 313.

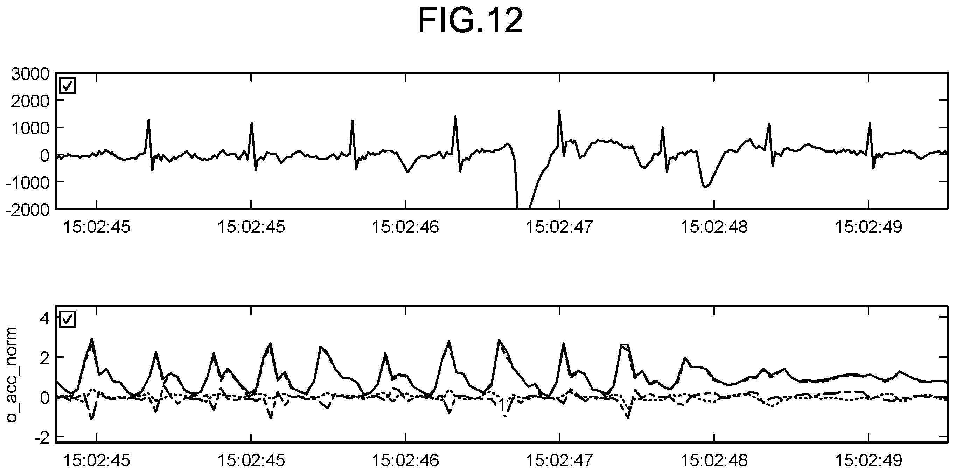

FIG. 12 is a diagram illustrating electrocardiogram waveform data acquired by Transmitter 01 in Comparative Example 1. As a result of comparing the electrocardiogram waveform data in Comparative Example 1 to the electrocardiogram waveform data in Example 1, it was found that noise in Comparative Example 1 was more intense than noise in Example 1, and hence Comparative Example 1 was more affected by body movements.

### (Comparative Example 2)

In Comparative Example 2, while an electrode and a belt were disposed in parallel with each other like Example 1, there was provided a belt in which a structure having the electrode disposed therein was not made of a textile, but made of a non-slip rubber molded article. In the same manner as in Example 1 and Comparative Example 1, wearer jogged, took a rest, and went up and down the stairs.

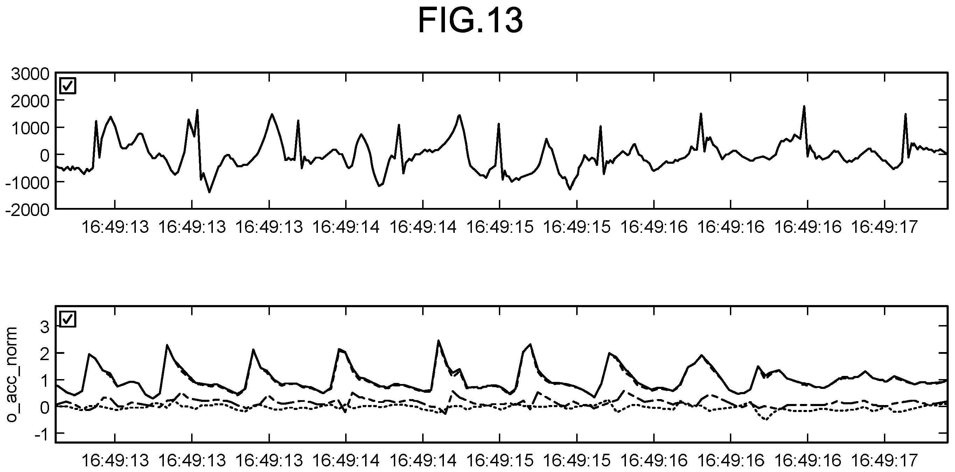

FIG. 13 is a diagram illustrating electrocardiogram waveform data acquired by Transmitter 01 in Comparative Example 2. In Comparative Example 2, before exercise, a rubber molded article was less likely to slip on the skin and noise generation did not occur, but sweat during jogging was accumulated on a surface of the rubber molded article, and therefore, the rubber molded article became slippery and was easily displaced from the skin. As a result, it was found that, like Comparative Example 1, noise caused by body movements was much mixed in the data when the wearer went up and down the stairs.

### Reference Signs List

1, 1A, 1B, 311 elastic belt
2, 2A, 312 connector
3, 3A, 3B, 3C textile structure
4, 4A, 313 electrode
5 waterproof cover
11 belt joint
31, 31A, 31B, 31C main body
32, 32A, 32B cushioning member
33, 33A, 33B non-slip textile
34 belt loop
35, 41A button
101 to 106, 301 biosignal acquiring tool
201 electronic device

## Claims

1. A biosignal acquiring tool (101,102,103,104,105,106, 301), comprising:
a textile structure (3,3A,3B,3C) in which a non-slip textile (33,33A,33B) is disposed;
an electrode (4,4A,313) positioned on a back side of the textile structure(3,3A,3B,3C);
a connector (2,2A,312) configured to connect an electronic device (201) configured to acquire a signal from the electrode (4,4A,313); and
an elastic belt (1,1A,1B,311) capable of being stretched independently of the textile structure (3,3A,3B,3C), the elastic belt (1,1A,1B,311) being disposed in parallel with the textile structure (3,3A,3B,3C) to face a front side of the textile structure (3,3A,3B,3C) that is different from a back side on which the electrode (4,4A,313) is positioned, the elastic belt (1,1A,1B,311) being coupled to the textile structure (3,3A,3B,3C) at a portion different from a portion at which the electrode (4,4A,313) is positioned and at a portion different from surroundings of the portion, wherein the textile structure (3,3A,3B,3C) is capable of being displaced within a range of 2 mm or more and 10 mm or less in a width direction of the elastic belt (1,1A,1B,311),
and wherein the back side corresponds to a side closer to a wearer's skin in use.

2. The biosignal acquiring tool (101,102,103,104,105,106, 301) according to claim 1, wherein the non-slip textile (33,33A,33B) is a knitted fabric including an elastic fiber and an inelastic fiber, and has a surface occupancy of the elastic fiber being 30% or more and 70% or less.

3. The biosignal acquiring tool (101,102,103,104,105, 106,301) according to claim 1 or 2, wherein
the textile structure (3,3A,3B,3C) has a tubular shape, and
the elastic belt (1,1A,1B,311) is configured to pass through inside of the tubular shape.

4. The biosignal acquiring tool (101,102,103,104, 105,106,301) according to any one of claims 1 to 3, wherein the textile structure (3,3A,3B,3C) includes a cushioning member (32,32A,32B) having a thickness of 2 to 10 mm, the cushioning member (32,32A,32B) being disposed outside the electrode (4,4A,313) and inside the elastic belt (1,1A,1B,311) disposed in parallel with the textile structure (3,3A,3B,3C).

5. The biosignal acquiring tool (101,102,103,104,105,106, 301) according to any one of claims 1 to 4, wherein the textile structure (3,3A,3B,3C) and the elastic belt (1,1A,1B, 311) are integrally fixed between the connector (2,2A,312) and the electrode (4,4A,313).

6. The biosignal acquiring tool (101,102,103,104,105,106, 301) according to any one of claims 1 to 5, wherein the electrode (4,4A,313) is removable, and fixed to the textile structure (3,3A,3B,3C) with a metal hook.

7. The biosignal acquiring tool (101,102,103,104,105, 106,301) according to any one of claims 1 to 6, wherein the electrode (4,4A,313) is configured with an electrical conductive textile including a nanofiber.

8. The biosignal acquiring tool (101,102,103,104,105,106, 301) according to any one of claims 1 to 7, further comprising a waterproof cover (5) configured to cover an upper portion and a front-side surface of the electronic device (201) from a back-side surface of the connector (2,2A,312), with the electronic device (201) being attached to the connector (2,2A,312).

## Patentansprüche

1. Biosignalerfassungswerkzeug (101, 102, 103, 104, 105, 106, 301), das Folgendes umfasst:
eine Textilstruktur (3, 3A, 3B, 3C), in der ein rutschfestes Textil (33, 33A, 33B) angeordnet ist;
eine Elektrode (4, 4A, 313), die auf einer Rückseite der Textilstruktur (3, 3A, 3B, 3C) angeordnet ist;
einen Verbinder (2, 2A, 312), der dazu ausgelegt ist, eine elektronische Vorrichtung (201) zu verbinden, die dazu ausgelegt ist, ein Signal von der Elektrode (4, 4A, 313) zu erfassen; und
einen elastischen Gürtel (1, 1A, 1B, 311), der in der Lage ist, unabhängig von der Textilstruktur (3, 3A, 3B, 3C) gedehnt zu werden, wobei der elastische Gürtel (1, 1A, 1B, 311) parallel zur Textilstruktur (3, 3A, 3B, 3C) angeordnet ist, um einer Vorderseite der Textilstruktur (3, 3A, 3B, 3C) zugewandt zu sein, welche eine andere ist als die Rückseite, auf der die Elektrode (4, 4A, 313) angeordnet ist, wobei der elastische Gürtel (1, 1A, 1B, 311) in einem Abschnitt, der sich von einem Abschnitt unterscheidet, in dem die Elektrode (4, 4A, 313) angeordnet ist, und in einem Abschnitt, der sich von einer Umgebung des Abschnitts unterscheidet, mit der Textilstruktur (3, 3A, 3B, 3C) gekoppelt ist, wobei die Textilstruktur (3, 3A, 3B, 3C) in der Lage ist, innerhalb eines Bereichs von 2 mm oder mehr und 10 mm oder weniger in einer Breitenrichtung des elastischen Gürtels (1, 1A, 1B, 311) verschoben zu werden,
und wobei die Rückseite einer Seite entspricht, die im Gebrauch näher zur Haut eines Benutzers liegt.

2. Biosignalerfassungswerkzeug (101, 102, 103, 104, 105, 106, 301) nach Anspruch 1, wobei das rutschfeste Textil (33, 33A, 33B) ein Gestrick ist, das eine elastische Faser und eine nichtelastische Faser umfasst und eine Flächenbelegung der elastischen Faser von 30 % oder mehr und 70 % oder weniger aufweist.

3. Biosignalerfassungswerkzeug (101, 102, 103, 104, 105, 106, 301) nach Anspruch 1 oder 2, wobei
die Textilstruktur (3, 3A, 3B, 3C) eine Schlauchform aufweist und
der elastische Gürtel (1, 1A, 1B, 311) dazu ausgelegt ist, durch das Innere der Schlauchform hindurch zu verlaufen.

4. Biosignalerfassungswerkzeug (101, 102, 103, 104, 105, 106, 301) nach einem der Ansprüche 1 bis 3, wobei die Textilstruktur (3, 3A, 3B, 3C) ein Polsterungselement (32, 32A, 32B) umfasst, das eine Dicke von 2 bis 10 mm aufweist, wobei das Polsterungselement (32, 32A, 32B) außerhalb der Elektrode (4, 4A, 313) und innerhalb des elastischen Gürtels (1, 1A, 1B, 311) angeordnet ist, der parallel zur Textilstruktur (3, 3A, 3B, 3C) angeordnet ist.

5. Biosignalerfassungswerkzeug (101, 102, 103, 104, 105, 106, 301) nach einem der Ansprüche 1 bis 4, wobei die Textilstruktur (3, 3A, 3B, 3C) und der elastische Gürtel (1, 1A, 1B, 311) einstückig zwischen dem Verbinder (2, 2A, 312) und der Elektrode (4, 4A, 313) befestigt sind.

6. Biosignalerfassungswerkzeug (101, 102, 103, 104, 105, 106, 301) nach einem der Ansprüche 1 bis 5, wobei die Elektrode (4, 4A, 313) entfernbar und mit einem Metallhaken auf der Textilstruktur (3, 3A, 3B, 3C) befestigt ist.

7. Biosignalerfassungswerkzeug (101, 102, 103, 104, 105, 106, 301) nach einem der Ansprüche 1 bis 6, wobei die Elektrode (4, 4A, 313) mit einem elektrisch leitfähigen Textil ausgelegt ist, das eine Nanofaser umfasst.

8. Biosignalerfassungswerkzeug (101, 102, 103, 104, 105, 106, 301), nach einem der Ansprüche 1 bis 7, ferner umfassend eine wasserfeste Abdeckung (5), die dazu ausgelegt ist, einen oberen Abschnitt und eine Vorderseitenfläche der elektronischen Vorrichtung (201) von einer Rückseitenfläche des Anschlusses (2, 2A, 312) aus abzudecken, wobei die elektronische Vorrichtung (201) an dem Verbinder (2, 2A, 312) befestigt ist.

## Revendications

1. Outil d'acquisition de signal biologique (101, 102, 103, 104, 105, 106, 301), comprenant :
une structure de textile (3, 3A, 3B, 3C) dans laquelle un textile antidérapant (33, 33A, 33B) est disposé ;
une électrode (4,4A, 313) positionnée sur un côté arrière de la structure de textile (3, 3A, 3B, 3C) ;
un connecteur (2,2A, 312) configuré pour connecter un dispositif électronique (201) configuré pour acquérir un signal en provenance de l'électrode (4, 4A, 313) ; et
une ceinture élastique (1,1A, 1B, 311) pouvant être étirée indépendamment de la structure de textile (3,3A, 3B, 3C), la ceinture élastique (1,1A, 1B, 311) étant disposée parallèlement à la structure de textile (3, 3A, 3B, 3C) pour faire face à un côté avant de la structure de textile (3, 3A, 3B, 3C) qui est différent d'un côté arrière sur lequel l'électrode (4, 4A, 313) est positionnée, la ceinture élastique (1,1A, 1B, 311) étant couplée à la structure de textile (3, 3A, 3B, 3C) au niveau d'une partie différente d'une partie sur laquelle l'électrode (4, 4A, 313) est positionnée et au niveau d'une partie différente de l'environnement de la partie, dans lequel la structure de textile (3, 3A, 3B, 3C) peut être déplacée dans une plage de 2 mm ou plus et de 10 mm ou moins dans une direction de largeur de la ceinture élastique (1, 1A, 1B, 311),
et dans lequel le côté arrière correspond à un côté plus proche de la peau d'un porteur en utilisation.

2. Outil d'acquisition de signal biologique (101, 102, 103, 104, 105, 106, 301) selon la revendication 1, dans lequel le textile antidérapant (33, 33A, 33B) est un tissu tricoté incluant une fibre élastique et une fibre inélastique, et présente une occupation de surface de la fibre élastique de 30 % ou plus et de 70 % ou moins.

3. Outil d'acquisition de signal biologique (101, 102, 103, 104, 105, 106, 301) selon la revendication 1 ou 2, dans lequel
la structure de textile (3, 3A, 3B, 3C) présente une forme tubulaire, et
la ceinture élastique (1, 1A, 1B, 311) est configurée pour passer à travers l'intérieur de la forme tubulaire.

4. Outil d'acquisition de signal biologique (101, 102, 103, 104, 105, 106, 301) selon l'une quelconque des revendications 1 à 3, dans lequel la structure de textile (3, 3A, 3B, 3C) inclut un élément d'amortissement (32, 32A, 32B) présentant une épaisseur de 2 à 10 mm, l'élément d'amortissement (32, 32A, 32B) étant disposé à l'extérieur de l'électrode (4, 4A, 313) et à l'intérieur de la ceinture élastique (1, 1A, 1B, 311) disposée en parallèle avec la structure de textile (3, 3A, 3B, 3C).

5. Outil d'acquisition de signal biologique (101, 102, 103, 104, 105, 106, 301) selon l'une quelconque des revendications 1 à 4, dans lequel la structure de textile (3, 3A, 3B, 3C) et la ceinture élastique (1, 1A, 1B, 311) sont fixées d'un seul tenant entre le connecteur (2, 2A, 312) et l'électrode (4, 4A, 313).

6. Outil d'acquisition de signal biologique (101, 102, 103, 104, 105, 106, 301) selon l'une quelconque des revendications 1 à 5, dans lequel l'électrode (4, 4A, 313) est amovible et fixée à la structure de textile (3, 3A, 3B, 3C) à l'aide d'un crochet métallique.

7. Outil d'acquisition de signal biologique (101, 102, 103, 104, 105, 106, 301) selon l'une quelconque des revendications 1 à 6, dans lequel l'électrode (4, 4A, 313) est configurée avec un textile conducteur électrique incluant une nanofibre.

8. Outil d'acquisition de signal biologique (101, 102, 103, 104, 105, 106, 301) selon l'une quelconque des revendications 1 à 7, comprenant en outre un couvercle étanche à l'eau (5) configuré pour recouvrir une partie supérieure et une surface de côté avant du dispositif électronique (201) à partir d'une surface de côté arrière du connecteur (2, 2A, 312), le dispositif électronique (201) étant fixé au connecteur (2, 2A, 312).
